(19) Europäisches Patentamt
European Patent Office
Office européen des brevets

(11) EP 3 541 356 B1

(12) **EUROPEAN PATENT SPECIFICATION**

(45) Date of publication and mention of the grant of the patent:
**22.04.2020 Bulletin 2020/17**

(21) Application number: **18708182.3**

(22) Date of filing: **22.02.2018**

(51) Int Cl.:
*A61K 8/37* (2006.01) *A61K 8/39* (2006.01)
*A61K 8/41* (2006.01) *A61K 8/42* (2006.01)
*A61K 8/44* (2006.01) *A61K 8/46* (2006.01)
*A61K 8/86* (2006.01) *A61Q 19/10* (2006.01)
*A61K 8/92* (2006.01) *A61K 8/04* (2006.01)

(86) International application number:
**PCT/GB2018/050455**

(87) International publication number:
**WO 2018/154298 (30.08.2018 Gazette 2018/35)**

(54) **FOAMABLE CLEANSING COMPOSITIONS**

SCHAUMBARE REINIGUNGSZUSAMMENSETZUNG

COMPOSITION NETTOYANTE MOUSSABLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.02.2017 GB 201702905**

(43) Date of publication of application:
**25.09.2019 Bulletin 2019/39**

(73) Proprietor: **Deb IP Limited**
**Denby, Derbyshire DE5 8JZ (GB)**

(72) Inventors:
- **KAMPS, Nicole**
**Denby**
**Derbyshire DE5 8JZ (GB)**
- **VEEGER, Marcel**
**Denby**
**Derbyshire DE5 8JZ (GB)**
- **THIEMANN, Astrid**
**Denby**
**Derbyshire DE5 8JZ (GB)**
- **FELLOWS, Caroline**
**Denby**
**Derbyshire DE5 8JZ (GB)**

(74) Representative: **Elsy, David**
**Withers & Rogers LLP**
**4 More London Riverside**
**London SE1 2AU (GB)**

(56) References cited:
**WO-A1-2012/021130 WO-A1-2014/123916**
**WO-A1-2016/179503 DE-C1- 19 934 836**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The invention relates to foamable skin and hand cleansing compositions, especially to those adapted to be used in combination with inverted air pumps for producing a foamed product.

Liquid skin and hand cleansing formulations are generally known in the art. They are conventionally provided in containers that are poured or have pumps to pump the liquid compositions onto the skin to be cleaned. Such liquid soaps often have very good cleaning efficiency.

Formulations which are foamed before being placed on the skin or hands are also generally known in the art. Foams tend to be much easier to spread than the corresponding liquid and in addition there is much less waste due to splashing or run off since the foam has a much higher surface tension than the liquid. One problem associated with foams compared with liquid soaps is they tend to have a lower cleaning efficiency. However, foams have a much higher surface area than unfoamed liquid, so if the cleaning efficiency of the foam can be improved, it is possible to produce foams with the same cleaning power, as obtained with un-foamed liquid, but which require much less of the initial liquid to be used.

A number of cleansing formulations use a combination of oils and emulsifiers to assist in the removal of dirt from the skin or hands to be treated. The Applicant decided to try and produce such an oil based cleansing formulation which is able to be foamed.

Foaming pumps are generally known in the art. They are usually upright, in which the foamable liquid is placed within a container such as a bottle. A pumping unit is attached to the top of the bottle and comprises a dispenser which is pumped downwards towards the bottle and forces air through the liquid to cause foam to be dispensed from the dispenser onto the skin or hands. When the Applicant tried to produce formulations with such upright pumps, they were able to produce suitable foams by the addition of conventional washing additives such as lauramine oxide and betaines.

Inverted pumps are also known in which the foam dispenser is provided at the bottom of a container within a dispenser placed on, for example, the wall of a room. Such dispensers have the advantage that the foamable liquid does not need to be pumped from the bottom of a storage container by a tube extending down to the bottom of the container. This removes a problem associated with such upright containers that, as the liquid is depleted, greater force must be exerted in the pumping procedure in order to raise the liquid from the bottom of the container during dispensing of the liquid. Inverted foam dispensers are shown in, for example, WO 99/49769 and WO 2014/138958. Such dispensers foam from the base of the dispenser, rather than a top pump foam dispenser. The foaming element and/or pump are typically at the base of a container. Foam is expelled from the base of the dispenser on activating a pump, such as a manually operated pump, to expel a foamable composition through a foaming component or element of the dispenser. Patent document WO 2012/021130 discloses aqueous, foaming cleanser compositions.

However, when the Applicant tried to use the oil and emulsifier-based cleansing formulations, in combination with conventional washing additives, they found that they were unable to obtain satisfactory quality foam from the inverted dispensers, despite the fact that the same formulations worked with conventional upright dispensers. A wide range of different additives were tried, until the Applicant unexpectedly found a class of additives which allowed a formulation to be produced, which could be used in both inverted and upright foam dispensers.

The solution to this problem was not immediately apparent as formulations comprising the washing additive without the oil were still found to have problems being dispensed from inverted dispensers. It was only when the washing additive was combined with the oil and emulsifier that the problem with the inverted dispenser was resolved.

The class of additives used to overcome the problem of using the inverted foam dispensers are known as fatty acid glucamides. These have been previously used in washing and cleaning formulations and in the cosmetics industry as surfactants. There are described generally in EP 1072615 and commercially available from Clariant GmbH, Germany under the trade name Glucotain™. They are typically produced from sugars, such as D-glucose via reaction with methylamine and hydrogenation to open out the structure of the glucose to produce glucamine. This is then reacted with, for example, a fatty acid methylester or triglyceride to produce the glucamide.

Accordingly, the invention provides a foamable skin and hand cleansing composition comprising the following components, in each cased based on the total composition of the cleaning composition:

a) 0.1 to 5% by weight of at least one emulsifier;
b) 0.5 to 7% by weight of at least one oil;
c) 0 to 10% by weight of at least one hydrophobic emollient;
d) 5 to 20% by weight of at least one surfactant used as a soiling remover;
e) 0.5 to 12% by weight of at least one fatty acid glucamide
f) 1 to 8% by weight of at least one surfactant used as a foamer
g) 0 to 1% by weight of at least one preservative
h) 0 to 1% by weight of a pH modifier
i) 0 to 10% by weight of one or more auxiliaries or additives

j) water to make up 100% by weight.

The composition typically comprises 0.7 to 10%, or 1 to 5% by weight of at least one fatty acid glucamide (e). Fatty acid glucamide (e) typically has a general formula:

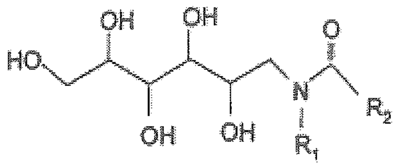

(1)

where:

$R^1$ = H or a C1 to C4 alkyl, most typically H or $-CH_3$, especially $-CH_3$
$R^2$ = $C_6$ to $C_{20}$ linear or branched hydrocarbon residue, most typically $C_8$ to $C_{18}$ linear or unbranched hydrocarbon residue. The hydrocarbon residue may be saturated or unsaturated.

Mixtures of the glucoamines may be used. For example, the glucamide may be a mixture of capryloyl/caproyl methyl glucamide; lauroyl/myristoyl methyl glucamide; or cocoyl methyl glucamide. Mixtures of these may also be used, for example, a mixture of cocoyl methyl glucamide and capryloyl/caproyl methyl glucamide may be used. Most typically the glucoamide is cocoyl methyl glucamide comprising fatty acid residues from coconut oil.

The oil (b) may be an alkyl fatty acid ester, most typically an isopropyl fatty acid ester or an ethylhexyl fatty acid ester. The fatty acid moiety of the ester may be a $C_6$ to $C_{20}$, such as $C_{12}$ to $C_{15}$ saturated or unsaturated fatty acid. These include, for example, saturated fatty acids and include myristic acid, palmitic acid and stearic acid and mixtures of fatty acids such as derived from rape seed oil. Most typically the alkyl moiety comprises 1-5 carbons including methyl, ethyl, propyl, butyl, isopropyl. pentyl or ethylhexyl, or is an alkyl benzoate, such as $C_{12}$ to $C_{15}$ alkyl benzoate. More typically the alkyl is isopropyl or ethylhexyl, especially isopropyl. Mixtures of two or more oils may be used. Most typically the oil is isopropyl myristate or a mixture of isopropyl myristate and isopropyl palmitate. Ethylhexyl stearate may be used. The oils are typically substantially clear oils. The composition may comprise 1 to 5% by weight, or 3 to 5% by weight of the oil(s).

The emulsifier (a) may be selected from those generally known in the art including PEG-18 castor dioleate, polyglycerin 4-caproate, hydrogenated castor oil, PEG-10 glyceryl dioleate and gemini surfactants and mixtures thereof. Gemini surfactants are a family of surfactant molecules possessing more than one hydrophobic tail and hydrophilic head group. Gemini surfactants usually have better surface-active properties than corresponding conventional surfactants of equal chain length. For example, they have the hydrophobic to hydrophilic head groups connected to one another via a spacer. These are described in, for example, a review article by S. K. Hate and S.P. Moulik, Current Science, Vol 82(9) (2002) 1101-1111.

Most typically the emulsifier is PEG-18 castor oil dioleate, a Gemini surfactant or PEG 10-dioleate, especially PEG-10 dioleate or a Gemini surfactant.

Other emulsifiers that can be used are glycereth-7 caprylate/caprate (Emanon Ev-E),

The composition may comprise 1 to 5% by weight of emulsifier (a) or 2 to 3 % by weight.

The emollient (c) may be any suitable emollient that is also known as solubilizer known in the art, but is typically PEG-7 glyceryl cocoate. Other suitable emollients include polyglycerine 4 caprate, glycereth-2 cocoate, or PEG-6 caprylic/capric glycerides.

The emollients typically have an HLB value of at least 10 and typically at least 12

They are typically polyol esters such as polyglyceryl partial esters or polyglycerol fatty acid esters particularly preferred according to the invention are, for example, polyglyceryl-3 caprate or polyglyceryl-4 caprate, which are available from Degussa under the names TEGOSOFT® PC31 and TEGOSOFT® PC41.

Polyol esters for the purposes of the present invention are furthermore polyethylene glycol esters, such as, for example, PEG-7 glyceryl cocoate (discussed above), which is available from Croda Chemicals Europe Ltd. under the name Glycerox HE.

The composition typically comprises 8 to 15% by weight on anionic surfactant (d). The anionic surfactant may, for example, be an alkyl sulfate, such as sodium or ammonium lauryl sulfate or alkyl ether sulfate such as sodium laureth sulfate. Other suitable surfactants include Sodium alpha olefine sulfonate, N-cocoyl glutamic acid sodium salts, alkyl fatty acid isethionates, alkyl hydroxysultaine, alkylamidopropyl betaine, N-cocoyl glycine sodium salt, sulfonated and sulphated castor oils.

2-3% of foamer (f) may be used. Foamers are generally known in the art. This is typically not a silicon-containing

foamer as they have been found to change the ability to paint surfaces in the automotive industry. They include, for example, cocoamidopropyl betaine, capryl/capamidopropyl betaine fatty alcohol ether carboxylic acids and carboxylates, alkanol amides and amine oxides, ethoxylated alkyl sulfosuccinates.

Up to 1% by weight of one or more preservatives (g) may be used. These are generally known in the art and include, for example, 1, 2-hexane dial, caprylyl glycol and tropolone. More typically sodium benzoate is used. The pH of the formulation may be adjusted by the addition of more or more pH modifiers, such as citric acid.

0-10% by weight, more typically 2-8% or 3-7% by weight of one or more auxiliaries or additives (i) may be used in the formulation. These are generally known to the person skilled in the art. They may include, for example, consistency regulators, fragrances, thickeners such as polymers, inorganic and organic UV photo protective filters, pigments, antioxidants, plant extracts, stabilisers and humectants. 0-1%, more typically 0-1-0.8% of a fragrance may be used. This improves the smell of the composition. Such fragrances are generally known in the art..

Typically no additional auxiliaries are added to the formulation.

Water is used to make up the composition to 100% by weight.

Typically the composition comprises 7-20% by total weight of the composition. Washing active substances is the combined weight of the surfactant, alkyl glucamide and foamer. (D, E, F.)

More typically washing additives contain 8-16 or 10-14% by weight washing additives.

The use of the compositions for skin and/or hand washing and methods of using the formulations for compositions for skin and/or hand washing are also provided.

The invention will now be described by way of example only, with reference to the following figure:

**Figure 1** shows the mean cleaning efficiencies of two foams made in accordance with the invention (Estesol Foam Pure and Estesol Foam) in comparison with Stephalen™ Flash Foam and Refresh™ Azure Foam, and in comparison with two liquid cleansers; GojoEco Soy™ and Estesol™ Mild Wash.

**Foam Production**

The formulations described below were tested using upright foam pumps commercially available from Albea (Le Cignac, Gennevilliers, France). The inverted pumps (Optidose) are commercially available wall mounted dispensers obtainable from Deb Limited, United Kingdom.

**Cleansing Performance**

Cleansing performance was typically carried out using the following method:

The test model of the hand washing test with standardised soiling or paint gives information about the cleaning effect of the products to be tested. For relevance in practice, it is necessary that all subjects have a characteristic skin structure on the palms of the hands caused by manual work. Using one product at time, the following test is carried out in the morning and afternoon:

<u>Test Procedure With Water:</u>

0.5g of soiling (model soiling, practice soiling or paint) is distributed on the palm and the back of the hand and rubbed in

Leave to dry for 1 1/2 min

1.2g of cleansing composition are applied and rubbed in

1 ml of water is added and wash for 30 s

Add a further 1 ml of water and wash for 30 s

Rinse under cold running water

Visual assessment of the residual soiling (RS) on the back of the hand and the palm according to the scale, see below.

<u>Test Procedure Without Water:</u>

0.5 g of soiling (model soiling, practice soiling or paint) are distributed on the palm and on the back of the hand and rubbed in

Leave to dry for 1 1/2 min

1.2 g of cleaning composition are applied and rubbed in

Using a cellulose paper, the soiling on the hand surfaces is removed together with the product

Visual assessment of the residual soiling (RS) on the back of the hand and the palm according to the scale, see below.

0=clean 5=no cleaning effect (graduation in steps of 0.5 possible).

The percentage cleaning effect is calculated according to the following formula:

$$\text{cleaning effect } [\%] = \frac{10 - (\overline{RS}_{palm} + \overline{RS}_{back})}{10} \cdot 100\%$$

$RS_{palm}$=rnean value of the residual soiling on palms of n measurement series (subjects).

$RS_{back}$=mean value of the residual soiling on backs of hands of n measurement series (subjects).

Since the deterioration of the cleaning effect has a broader variation range, an absolute deviation of 5% between two measurement series is allowable.

Composition of a suitable model soiling:

| | |
|---|---|
| Flame soot: | 5.42% |
| Iron oxide ($Fe_2O_3$): | 0.72% |

**Comparative Example 1**

The prior art formulation Sasol number 11574.

| **Formulation Sasol No. 11574 Mild and Caring Shower Foam** | | Z1 |
|---|---|---|
| **raw materials** | **INCI** | **%** |
| Marlowet CG | PEG-18 Castor Oil Dioleate | 1.00 |
| Marlinat 242/90 MC | MIPA Laureth Sulfate (and) Propylen Glycol | 12.00 |
| Sunflower Oil | Helianthus Annus Seed Oil | 1.00 |
| Cosmacol ELI | C12-13 Alkyl Lactate | 0.30 |
| Antil 200 | PEG 200 Hydrogenated Glyceryl Palmate (and) PEG-7 Glyceryl Cocoate | 0.20 |
| NaCl | Sodium Chloride | 2.00 |
| Cocoamidopropyl Betaine (38%active) | Cocoamidopropylbetaine | 5.00 |
| Preservative | | q.s. |
| Lactic acid | Lactic acid | q.s. |
| Perfume | Parfum/ Fragrance | q.s. |
| D-Panthenol 75 L | Panthenol | q.s. |
| Water | Water | add to 100 |
| WAS content in % | | 12.70 |
| Total | | 100.000 |
| Cleansing Performance | | 49% |

(continued)

| Formulation Sasol No. 11574 Mild and Caring Shower Foam | | Z1 |
|---|---|---|
| **raw materials** | **INCI** | **%** |
| Foam Quality Optidose 7:1 air/product ratio upside down | | - |
| Foam Quality Albea Pump 7:1 air/product ratio bottom up | | ++ |

Sasol Formulation 11574 is a mild and caring shower foam composition, comprising oil and is similar to the intended final product the inventors were trying to identify. Using the conventional upright Albea foam, it was possible to foam the formulation. However, the inverted Optidose pump was unable to produce foam.

**Comparative Example 2**

| Formulation Clariant All/4063 Luxurious Foam Essence Facial Cleanser | | Z2 |
|---|---|---|
| **raw materials** | **INCI** | % |
| Genapol LRO liq. | Sodium Laureth Sulfate | 14.800 |
| GlucoTain Care | Cocoyl Methyl Glucamide | 5.000 |
| GlucoTain Clear | Capryloyl/Caproyl Methyl Glucamide | 2.000 |
| NaCl | Sodium Chloride | 2.000 |
| Preservative | Sodium Benzoate | q.s. |
| Citric Acid 50% | Citric Acid | q.s. |
| Perfume | Parfum/ Fragrance | q.s. |
| Water | Water | add to 100 |
| WAS content in % | | 7.99 |
| Total | | 100.000 |
| Cleansing Performance | | 55% |
| Foam Quality Optidose 7:1 air/product ratio upside down | | - |
| Foam Quality Albea Pump 7:1 air/product ratio bottom up | | +++ |

Comparative Examples 2 shows a non-oil based cleanser, containing fatty acid alkyl glucamides. Again, that formulation was able to be foamed using upright foamers, but was unable to produce foams using inverted foaming devices and it has got also not a suitable cleansing performance.

On identifying the problem with using inverted foamers, the Applicant tried a number of different types of additives to try and produce a satisfactory composition that produced foaming in both the uprights and inverted pumps. These included:

Nonionics like Fatty alcohol ethoxylates such as Polyethylene glycol ethers of tridecyl alcohol, which have a good cleaning efficacy but inhibit the foam quality. Various trials with fatty alcohol ethoxylates to stabilise the foam failed.

To improve the foaming in combination with the cleansing properties. The Applicant also tried laureth-6 carboxylic acid and MIPA laureth sulfate. Alkylpolyglucose, disodium laureth sulfosuccinate, laureth-10, sodium cocoamphopropionate and N,N-dimethyl 9-decenamide were tried as alternatives but also failed .

A change from polyethylene glycol ethers to methyl glucamides (such as capryloyl/caproyl methyl glucamide, lauroyl/myristoyl methyl glucamide, cocoyl methyl glucamide) showed at least the beneficial effect while foaming and giving a good cleansing performance.

| raw materials | INCI | Aa1 [%] | Bb1 [%] | Bb2 [%] | Bb3 [%] | Cc1 [%] | Cc2 [%] | Dd1 [%] | Dd2 [%] | Dd3 [%] | Ee1 [%] | Ee2 [%] | Ff1 [%] | Ff2 [%] | Ff3 [%] | Gg1 [%] | Gg2 [%] | Gg3 [%] | Hh1 [%] | Hh2 [%] | Hh3 [%] |
|---|---|---|---|---|---|---|---|---|---|---|---|---|---|---|---|---|---|---|---|---|---|
| Marlowet CG | PEG-18 Castor Oil Dioleate | 2 | 2 | 2 | 2 | 2 | 1,6 | 2 | 2 | 2 | 2 | 2 | 2 | 2 | 2 | 2 | 2 | 2 | 2 | 2 | 2 |
| Tegosoft M | Isopropyl Myristate | 2 | 2 | 2 | 2 | 2 | 2 | 2 | 2 | 2 | 2 | 2 | 2 | 2 | 2 | 2 | 2 | 2 | 2 | 2 | 2 |
| Tegosoft OS | Ethylhexyl Stearate | 3 | 3 | 3 | 3 | 3 | 3 | 3 | 3 | 3 | 3 | 3 | 3 | 3 | 3 | 3 | 3 | 3 | 3 | 3 | 3 |
| Tegosoft GC | PEG-7 Glyceryl Cocoate | 7 | 7 | 7 | 7 | 7 | 7 | 7 | 7 | 7 | 7 | 7 | 7 | 7 | 7 | 7 | 7 | 7 | 7 | 7 | 7 |
| SLES, preserved 28% | Sodium Laureth Sulfate | 30 | 30 | 30 | 30 | 25 | 32 | 30 | 30 | 30 | 30 | 20 | 30 | 30 | 30 | 30 | 30 | 30 | 30 | 30 | 30 |
| Tego Betain 810 35% | Capryl/Capamidopropyl Betaine | 5 | 5 | 5 | 5 | | 5 | 5 | 5 | 5 | | | 5 | 5 | 5 | 5 | 5 | 5 | 5 | 5 | 5 |
| Trideceth-7 | Trideceth-7 | 4 | 4 | 4 | 4 | 4 | 4 | 4 | 4 | 3 | 4 | 1 | 4 | 4 | 4 | 4 | 4 | 4 | 4 | 4 | 4 |
| Barlox 12 | Cocoamine Oxide | | 3 | 2 | 1 | | | | | | | | | | | | | | | | |
| Benecel F 50 | Hydroxypropylmethyl Cellulose | | | | | | | 0,3 | | | | | | | | | | | | | |
| Benecel E 50 | Hydroxypropylmethyl Cellulose | | | | | | | | 0,3 | | | | | | | | | | | | |
| Stepan Mild LSB 25% | Sodium Lauryl Sufoacetate/ Disodium Laureth Sulfosuccinate | | | | | | | | | 1 | | | | | | | | | | | |
| Mackamine LA 31% | Lauramine Oxide | | | | | | | | | | 10 | 10 | | | | | | | | | |
| 1,3-Butandiol rein | Butylene Glycol | | | | | | | | | | 3 | 3 | 3 | 7 | 10 | | | | | | |
| Antil 200 | PEG-200 Hydrogenated Glyceryl Palmate, PEG-7 Glyceryl Cocoate | | | | | | | | | | | | | | | 0,5 | 1 | 1,5 | | | |
| Tegosoft LSE 65 K soft | Sucrose Cocoate | | | | | | | | | | | | | | | | | | 1 | 2 | 3 |
| Natriumbenzoat Typ 2 | Sodium Benzoate | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. |
| Citric Acid | Citric Acid | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. |
| Water | Water | add to 100 | add to 100 | add to 100 | add to 100 | add to 100 | add to 100 | add to 100 | add to 100 | add to 100 | add to 100 | add to 100 | add to 100 | add to 100 | add to 100 | add to 100 | add to 100 | add to 100 | add to 100 | add to 100 | add to 100 |
| WAS content in % | | 14,15 | 17,15 | 16,15 | 15,15 | 11,00 | 14,71 | 14,15 | 14,15 | 13,40 | 15,50 | 9,70 | 14,15 | 14,15 | 14,15 | 14,65 | 15,15 | 15,65 | 15,15 | 16,15 | 17,15 |
| Total | | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 |
| Cleansing performance | | >75% | >75% | >75% | >75% | >75% | >75% | >75% | >75% | >75% | >75% | >75% | >75% | >75% | >75% | >75% | >75% | >75% | >75% | >75% | >75% |
| Foam Quality Optidose 7:1 air/product ratio upside down | | - | - | - | - | - | - | - | - | - | - | - | - | - | - | - | - | - | - | - | - |
| Foam Quality Albea Pump 7:1 air/product ratio bottom up | | +++ | +++ | +++ | +++ | +++ | +++ | +++ | +++ | +++ | +++ | +++ | +++ | +++ | +++ | +++ | +++ | +++ | +++ | +++ | +++ |

| raw materials | INCI | Ii1 [%] | Ii2 [%] | Ii3 [%] | Ii4 [%] | Jj1 [%] | Jj2 [%] | Jj3 [%] | Jj4 [%] | Kk1 [%] | Kk2 [%] | Ll1 [%] | Ll2 [%] | Mm1 [%] | Mm2 [%] | Mm3 [%] | Mm4 [%] | Nn1 [%] | Nn2 [%] | Nn3 [%] | Oo1 [%] | Oo2 [%] | Oo3 [%] | Pp1 [%] | Pp2 [%] | Pp3 [%] |
|---|---|---|---|---|---|---|---|---|---|---|---|---|---|---|---|---|---|---|---|---|---|---|---|---|---|---|
| Marlowat CG | PEG-18 Castor Oil Dioleate | 2 | 2 | 2 | 2 | 2 | 2 | 2 | 2 | 2 | 2 | 2 | 2 | 2 | 2 | 2 | 2 | 2 | 2 | 2 | 2 | 2 | 2 | 2 | 2 | 2 |
| Tegosoft M | Isopropyl Myristate | 2 | 2 | 2 | 5 | 2 | 2 | 2 | 2 | 2 | 2 | 2 | 2 | 2 | 2 | 2 | 2 | 2 | 2 | 2 | 2 | 2 | 2 | 2 | 2 | 2 |
| Tegosoft OS | Ethylhexyl Stearate | 2 | 1 | | | 3 | 3 | 3 | 3 | 3 | 3 | 3 | 3 | 3 | 3 | 3 | 3 | 3 | 3 | 3 | 3 | 3 | 3 | 3 | 3 | 3 |
| Tegosoft GC | PEG-7 Glyceryl Cocoate | 7 | 7 | 7 | 7 | 7 | 7 | 7 | 7 | 7 | 7 | 7 | 7 | 7 | 7 | 7 | 7 | 7 | 7 | 7 | 7 | 7 | 7 | 7 | 7 | 7 |
| SLES, preserved 28% | Sodium Laureth Sulfate | 30 | 30 | 30 | 30 | 20 | 15 | 20 | 10 | 30 | 30 | 30 | 30 | 30 | 30 | 30 | 30 | 30 | 30 | 30 | 30 | 30 | 30 | 30 | 30 | 30 |
| Tego Betain 810 35% | Capryl/Capamidopropyl Betaine | 6 | 7 | 8 | 5 | 15 | 20 | 5 | 5 | 5 | 5 | | 5 | 5 | 5 | 5 | 5 | 6 | 6 | 6 | 6 | 6 | 6 | 6 | 6 | 6 |
| Trideceth-7 | Trideceth-7 | 4 | 4 | 4 | 4 | 4 | 4 | 4 | 4 | 4 | 4 | 4 | 2 | 4 | 4 | 4 | 4 | 4 | 4 | 4 | 4 | 4 | 4 | 4 | 4 | 4 |
| Tegosoft LSE 65 K soft | Sucrose Cocoate | 3 | 3 | 3 | 3 | 3 | 3 | 3 | 3 | | | | | | | | | | | | | | | | | |
| Protelan LS 9011 | Sodium Lauroyl Sarcosinate | | | | | | | 10 | 20 | | | | | | | | | | | | | | | | | |
| Elfan AT 84 G | Sodium Cocoyl Isethionate | | | | | | | | | 1,5 | | | | | | | | | | | | | | | | |
| Hostapon SCI 65 C | Sodium Cocoyl Isethionate, Stearic Acid, Sodium Isethionate, Aqua | | | | | | | | | | 1,5 | | | | | | | | | | | | | | | |
| Rewoteric AM KSF 40 | Sodium Cocoamphopropionate | | | | | | | | | | | 5 | 2 | | | | | | | | | | | | | |
| Dermofeel Sensolv | Isoamyl Laurate | | | | | | | | | | | | | 1 | | | | | | | | | | | | |
| Glycerin | Glycerin | | | | | | | | | | | | | | 0,5 | 3 | 5 | | | | | | | | | |
| Polyglykol 300 | PEG-6 | | | | | | | | | | | | | | | | | 0,5 | | | | | | | | |
| Polyglykol 400 | PEG-8 | | | | | | | | | | | | | | | | | | 0,5 | | | | | | | |
| Polyglykol 600 | PEG-12 | | | | | | | | | | | | | | | | | | | 0,5 | 0,2 | 3 | 5 | | | |
| Propandiol 1,2 | Propylene Glycol | | | | | | | | | | | | | | | | | | | | | | | 2 | 5 | 10 |
| Natriumbenzoat Typ 2 | Sodium Benzoate | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. |
| Citric Acid | Citric Acid | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. | q.s. |
| Water | Water | add to 100 | add to 100 | add to 100 | add to 100 | add to 100 | add to 100 | add to 100 | add to 100 | add to 100 | add to 100 | add to 100 | add to 100 | add to 100 | add to 100 | add to 100 | add to 100 | add to 100 | add to 100 | add to 100 | add to 100 | add to 100 | add to 100 | add to 100 | add to 100 | add to 100 |
| WAS content in % | | 17,50 | 17,85 | 18,20 | 17,15 | 17,85 | 18,20 | 24,35 | 31,55 | 15,65 | 15,65 | 17,40 | 14,15 | 15,15 | 14,15 | 14,15 | 14,15 | 14,5 | 14,5 | 14,5 | 14,5 | 14,5 | 14,5 | 14,5 | 14,5 | 14,5 |
| Total | | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 |
| Cleansing performance | | >75% | >75% | >75% | >75% | >75% | >75% | >75% | >75% | >75% | >75% | >75% | >75% | >75% | >75% | >75% | >75% | >75% | >75% | >75% | >75% | >75% | >75% | >75% | >75% | >75% |
| Foam Quality Optidose 7:1 air/product ratio upside down | | - | - | - | - | - | - | - | - | - | - | - | - | - | - | - | - | - | - | - | - | - | - | - | - | - |
| Foam Quality Albea Pump 7:1 air/product ratio bottom up | | +++ | +++ | +++ | +++ | +++ | +++ | +++ | +++ | +++ | +++ | +++ | +++ | +++ | +++ | +++ | +++ | +++ | +++ | +++ | +++ | +++ | +++ | +++ | +++ | +++ |

| raw materials | INCI | Qq1 [%] | Rr1 [%] | Rr2 [%] | Ss1 [%] | Ss2 [%] | Ss3 [%] | Tt1 [%] | Tt2 [%] | Tt3 [%] | Tt4 [%] | Uu1 [%] | Uu2 [%] | Uu3 [%] | Vv1 [%] | Vv2 [%] | Vv1 [%] |
|---|---|---|---|---|---|---|---|---|---|---|---|---|---|---|---|---|---|
| Marlowet CG | PEG-18 Castor Oil Dioleate | 2 | 2 | 2 | 2 | 2 | 2 | 2 | 2 | 2 | 2 | 2 | 2 | 2 | 2 | 2 | 2 |
| Tegosoft M | Isopropyl Myristate | 2 | 2 | 2 | 2 | 2 | 2 | 2 | 2 | 2 | 2 | 2 | 2 | 2 | 2 | 2 | 2 |
| Tegosoft OS | Ethylhexyl Stearate | 3 | 3 | 3 | 3 | 3 | 3 | 3 | | | | 3 | 3 | 3 | | | |
| Tegosoft GC | PEG-7 Glyceryl Cocoate | 7 | 7 | 7 | 7 | 7 | 7 | | | | | 7 | 7 | 7 | 7 | 7 | 7 |
| SLES, preserved 28% | Sodium Laureth Sulfate | 30 | 30 | 30 | 30 | 30 | 30 | 30 | 30 | 30 | 15 | 30 | 30 | 30 | 30 | 30 | 30 |
| Tego Betain 810 35% | Capryl/Capamidopropyl Betaine | 5 | 5 | 5 | 5 | 5 | 5 | 5 | 8 | 5 | 20 | 5 | 5 | 5 | 8 | 8 | |
| Trideceth-7 | Trideceth-7 | | | | | | | | | | | | | | | | |
| Cremer Coore PG 4 Cocoate | Polyglyceryl-4 Cocoate | 4 | | | | | | | | | | | | | | | |
| Marlinat 242/90 M 90% | MIPA Laureth Sulfate, Propylene Glycol | | 4 | | | | | | | | | | | | | | |
| Akypo RLM 45 92% | Laureth-6 Carboxylic Acid | | | 4 | | | | | | | | | | | | | |
| Eucarol Age SS 45% | Disodium alkylpolyglukosesulfosuccinate | | | | 4 | | | | | | | | | | | | |
| Lavoral WW hochkonz. | Laureth-10 | | | | | 4 | | | | | | | | | | | |
| Rewoteric AM KSF 40 | Sodium Cocoamphopropionate | | | | | | 5 | | | | | | | | | | |
| Steposol MET 10 U 97% | N,N-dimethyl 9-decenamide | | | | | | | 4 | 4 | 4 | 4 | | | | | | |
| Tegosoft PC31 | Polyglyceryl-3 Caprate | | | | | | | | | 3 | 3 | | | | | | |
| Gluco Tain Clear 50% | Capryloyl/Caproyl Methyl Glucamide | | | | | | | | | | | 4 | | | 4 | 6 | |
| Gluco Tain Flex 35% | Lauroyl/Myristoyl Methyl Glucamide | | | | | | | | | | | | 4 | | | | |
| Gluco Tain Care 40% | Cocoyl Methyl Glucamide | | | | | | | | | | | | | 4 | 2 | | 4 |
| Betaine Coco Base 30% | Cocoamidopropylbetaine | | | | | | | | | | | | | | | | 8 |
| Natriumbenzoat Typ 2 | Sodium Benzoate | q.s. | q.s. | q.s. | q.s. | q.s. | q.s. | q.s. | q.s. | q.s. | q.s. | q.s. | q.s. | q.s. | q.s. | q.s. | q.s. |
| Citric Acid | Citric Acid | q.s. | q.s. | q.s. | q.s. | q.s. | q.s. | q.s. | q.s. | q.s. | q.s. | q.s. | q.s. | q.s. | q.s. | q.s. | q.s. |
| Water | Water | add to 100 | add to 100 | add to 100 | add to 100 | add to 100 | add to 100 | add to 100 | add to 100 | add to 100 | add to 100 | add to 100 | add to 100 | add to 100 | add to 100 | add to 100 | add to 100 |
| WAS content in % | | 14,15 | 13,75 | 13,83 | 11,95 | 14,15 | 15,15 | 14,03 | 15,08 | 14,03 | 15,08 | 12,15 | 11,55 | 11,75 | 14,00 | 14,20 | 12,40 |
| Total | | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 | 100.00 |
| Cleansing performance | | >70% | >70% | >70% | >70% | >70% | >70% | >70% | >70% | >70% | >70% | >70% | >70% | >70% | >70% | >70% | >70% |
| Foam Quality Optidose 7:1 air/product ratio upside down | | + | + | + | + | + | + | + | + | + | + | ++ | ++ | ++ | +++ | +++ | +++ |
| Foam Quality Albea Pump 7:1 air/product ratio bottom up | | +++ | +++ | +++ | +++ | +++ | +++ | +++ | +++ | +++ | +++ | +++ | +++ | +++ | +++ | +++ | +++ |

| raw materials | INCI | A1 [%] | A2 [%] | A3 [%] | A4 [%] | B1 [%] | B2 [%] | C1 [%] | C2 [%] | D1 [%] | D2 [%] | E1 [%] | E2 [%] | F1 [%] | F2 [%] |
|---|---|---|---|---|---|---|---|---|---|---|---|---|---|---|---|
| Marlowet CG | PEG-18 Castor Oil Dioleate | 2.000 | 2.000 | 2.000 | 2.000 | 2.000 | 2.000 | 2.000 | 2.000 | 2.000 | 1.000 | 2.000 | 2.000 | 2.000 | 2.000 |
| Tegosoft M | Isopropyl Myristate | 2.000 | 2.000 | 2.000 | 2.000 | 2.000 | 2.000 | 3.000 | 1.000 | 2.000 | 1.000 | 2.000 | 2.000 | | |
| Tegosoft P | Isopropyl Palmitate | | | | | | | | | | | | | 2.000 | 2.000 |
| Tegosoft GC | PEG-7 Glyceryl Cocoate | 7.000 | 7.000 | 7.000 | 7.000 | 7.000 | 7.000 | 7.000 | 7.000 | 7.000 | 7.000 | 7.000 | 7.000 | 7.000 | 7.000 |
| SLES, preserved 28% | Sodium Laureth Sulfate | 30.000 | 30.000 | 30.000 | 30.000 | 30.000 | 40.000 | 30.000 | 30.000 | 30.000 | 30.000 | 25.000 | 20.000 | 20.000 | 15.000 |
| Gluco Tain Care 55% | Cocoyl Methyl Glucamide | 1.000 | 2.000 | 3.000 | 5.000 | 10.000 | 10.000 | 3.000 | 3.000 | 10.000 | 10.000 | 3.000 | 3.000 | 3.000 | 3.000 |
| Betaine Coco Base (30%) | Cocoamidopropylbetaine | 10.000 | 9.000 | 9.000 | 9.000 | 9.000 | 9.000 | 9.000 | 9.000 | 9.000 | 9.000 | 15.000 | 20.000 | 10.000 | 5.000 |
| Natriumbenzoat Typ 2 | Sodium Benzoate | q.s. | q.s. | q.s. | q.s. | q.s. | q.s. | q.s. | q.s. | q.s. | q.s. | q.s. | q.s. | q.s. | q.s. |
| Citric Acid | Citric Acid | q.s | q.s | q.s | q.s | q.s | q.s. | q.s | q.s | q.s | q.s | q.s | q.s | q.s | q.s |
| Water | Water | add to 100 | add to 100 | add to 100 | add to 100 | add to 100 | add to 100 | add to 100 | add to 100 | add to 100 | add to 100 | add to 100 | add to 100 | add to 100 | add to 100 |
| WAS content in % | | 11.950 | 12.200 | 12.750 | 13.850 | 16.600 | 19.400 | 12.750 | 12.750 | 16.600 | 16.600 | 13.150 | 13.250 | 10.250 | 7.350 |
| Total | | 100.000 | 100.000 | 100.000 | 100.000 | 100.000 | 100.000 | 100.000 | 100.000 | 100.000 | 100.000 | 100.000 | 100.000 | 100.000 | 100.000 |
| Cleansing Performance | | >70% | >70% | >70% | >70% | >70% | > 70% | >70% | >70% | >70% | >70% | >70% | >70% | 65% | 61% |
| Foam Quality Optidose 7:1 air/product ratio upside down | | +++ | +++ | +++ | +++ | +++ | +++ | +++ | +++ | +++ | +++ | +++ | +++ | +++ | +++ |
| Foam Quality Albea Pump 7:1 air/product ratio bottom up | | +++ | +++ | +++ | +++ | +++ | +++ | +++ | +++ | +++ | +++ | +++ | +++ | +++ | +++ |

Results show that combining oils with fatty acid alkyl glucamides produces formulations that may be used in both upright and inverted foaming pumps. Using alternative formulations without alkyl glucamides produces formulations with lower foam quality with inverted pumps.

Figure 1 shows the mean cleaning efficacy of two formulations (Estesol Foam Pure and Estesol Foam) compared to two commercially available foaming compositions and two liquid cleansing compositions. The Estesol foams are both based on Formulation A3. Estesol foam has 0.2% by weight fragrance and 0.001% by weight of dye added with a reduction in the amount of water added.

Stephalen™ Fresh Foam is available from Peter Greven Physioderm GmbH, Euskirchen, Germany and comprises

Water,
Glycerin,
Sodium laureth sulfate,
PEG-7 glyceryl cocoate,
Cocamidopropyl betaine,
Sodium citrate,
Citric acid,
Sodium benzoate,
Potassium sorbate,
Perfume and
C.I. 42051

Refresh™ Azure Foam is available from Deb Limited, United Kingdom. It contains:

Aqua
Sodium laureth sulfate
Propylene glycol
Glycerin
PEG-7 glyceryl cocoate
Cocamidopropyl betaine
Parfum
Citric acid
2-Bromo-2-nitropropane-1,3-diol
Methylchloroisothiazolinone
Methylisothiazolinone
Magnesium nitrate
Magnesium chloride
CI 42090

Gojo™ Eco Soy™ is a liquid hand cleanser available from Gojo. It is an alcohol based hand cleanser and comprises:

Water
Ethoxylated branched C11-14, C13 rich alcohols
Fatty acids, soya Me esters
Polyoxyethylene tridecyl ether
Ethanol
Propylene glycol
Propan-2-ol

Estesol Mild Wash is available from Deb Limited, United Kingdom and comprises:

Aqua
Sodium Laureth Sulfate
Sodium Chloride
Disodium Laureth Sulfosuccinate
Laureth-2
Sulfated Castor Oil

Glycol Distearate
Steareth-4
Poylquaternium-7
Citric Acid
PEG-30 Glyceryl Cocoate
Sodium Benzoate

The figure shows that conventional foam products have lower cleaning efficacy than conventional liquid, unfoamed hand washes. The foaming compositions of the invention produce foams with a cleaning efficiency in excess of the conventional foaming compositions and in excess of or comparable to liquid cleaning compositions.

## Claims

1. A foamable skin and hand cleansing composition comprising the following components, in each case based on the total composition of the cleaning composition:

   a) 0.1 to 5% by weight of at least one emulsifier selected from PEG-18 castor dioleate, polyglycerine 4-caproate, hydrogenated castor oil, PEG-10 glyceryl dioleate, glycereth-7 caprylate/caprate, gemini surfactants and mixtures thereof;
   b) 0.5 to 7% by weight of at least one oil, especially an alkyl fatty acid ester;
   c) 0 to 10% by weight of at least one hydrophobic emollient; selected from polyol esters, and typically selected from PEG 7 glyceryl cocoate, polyglycerine 4 caprate, glycereth 2-cocoate, PEG-6 caprylic/capric glycerides, polyglyceryl 3 caprate, polyglyceryl 4-caprate and mixtures thereof;
   d) 5 to 20% by weight of at least one surfactant used as a soiling remover;
   e) 0.5 to 12% by weight of at least one fatty acid glucamide;
   f) 1 to 8% by weight of at least one surfactant used as a foamer;
   g) 0 to 1% by weight of at least one preservative;
   h) 0 to 1% by weight of a pH modifier;
   i) 0 to 10% by weight of one or more auxiliaries or additives;
   j) water to make up 100% by weight.

2. A composition according to claim 1, comprising 1 to 5.5% fatty acid glucamide (e).

3. A composition according to claims 1 or 2, wherein the at least one fatty acid glucamide has a general formula:

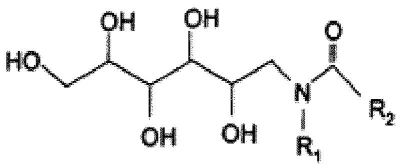

(1)

   Where:

   $R^1$ = H or a C1 to C4 alkyl
   $R^2$ = $C_6$ to $C_{20}$ linear or branched, saturated or unsaturated hydrocarbon residue, preferably wherein $R_1$ is-H or $-CH_3$, more preferably $=CH_3$.

4. A composition according to claim 3, wherein $R_2$ is a $C_8$ to $C_{18}$ linear branched or unbranched, saturated or unsaturated hydrocarbon residue.

5. A composition according to claims 1 to 4, wherein the glucamide (e) comprises a mixture selected from capryloyl/caproyl methyl glucamide, lauroyl/myristoyl methyl glucamide; and cocoyl methyl glucamide, most preferably wherein the glucamide (e) is cocoyl methyl glucamide.

6. A composition according to claims 1 to 5, wherein the oil (b an isopropyl or ethyl hexyl fatty acid ester or alkyl benzoate, preferably wherein the oil (b) is selected from ethylhexyl stearate, isopropyl myristate, isopropyl palmitate,

isopropyl stearate and isopropyl esters of rape seed oil or mixtures thereof.

**7.** A composition according to claims 1 to 6, wherein the composition comprises 1 to 3% by weight of the oil (b).

**8.** A composition according to claim 1, wherein the foamer is selected from cocamidopropyl betaine, capryl/capamido propyl betaine, fatty alcohol ether carboxylic acids and carboxylates, alkanol amides, amine oxides and ethoxylated alkyl sulfosuccinates.

**9.** A composition according to claims 1 to 8, comprising 1 to 3% by weight of the emulsifier (a).

**10.** A composition according to claims 1 to 9, comprising PEG-7 glyceryl cocoate, polyglyceryl -3-caprate, polyglycerine 4 caprate, glycereth-2 cocoate, or PEG-6 caprylic/capric glycerides
as the emollient (c).

**11.** A composition according to claims 1 to 10, wherein the composition comprises 8 to 15% anionic surfactant (d).

**12.** A composition according to claims 1 to 11, comprising 2 to 5% by weight of foamer (f).

**13.** A composition according to claims 1 to 12, wherein the washing active substances comprise 7-20% of the total weight of the composition.

**14.** Use of a composition according to claims 1 to 13 for skin and/or hand washing.

**15.** An inverted foam dispenser comprising a composition according to claims 1 to 14.

**16.** A method of producing a foam, comprising providing a composition according to claims 1 to 14 in an inverted foam dispenser and operating the dispenser to generate the foam.

## Patentansprüche

**1.** Schäumbare Haut- und Handreinigungszusammensetzung, umfassend die folgenden Bestandteile, jeweils bezogen auf die Gesamtzusammensetzung der Reinigungszusammensetzung:

a) 0,1 bis 5 Gew.-% mindestens eines Emulgators, ausgewählt aus PEG-18-Rizinusöldioleat, Polyglycerin-4-Caproat, hydriertem Rizinusöl, PEG-10-Glyceryldioleat, Glycereth-7-Caprylat/Caprinat, Geminitensiden und deren Mischungen;
b) 0,5 bis 7 Gew.-% mindestens eines Öls, insbesondere eines Alkylfettsäureesters;
c) 0 bis 10 Gew.-% mindestens eines hydrophoben Erweichungsmittels, ausgewählt aus Polyolestern und typischerweise ausgewählt aus PEG 7-Glycerylcocoat, Polyglycerin-4-Caprinat, Glycereth-2-Cocoat, PEG-6-Caprylat/Caprin-Glyceriden, Polyglyceryl-3-Caprinat, Polyglyceryl-4-Caprinat und Mischungen davon;
d) 5 bis 20 Gew.-% mindestens eines als Schmutzentferner verwendeten Tensids;
e) 0,5 bis 12 Gew.-% mindestens eines Fettsäureglucamids;
f) 1 bis 8 Gew.-% mindestens eines als Schaumbildner verwendeten Tensids;
g) 0 bis 1 Gew.-% mindestens eines Konservierungsmittels;
h) 0 bis 1 Gew.-% eines pH-Modifizierungsmittels;
i) 0 bis 10 Gew.-% eines oder mehrerer Hilfs- oder Zusatzstoffe;
j) Wasser, auf 100 Gew.-% aufgefüllt.

**2.** Zusammensetzung nach Anspruch 1, umfassend 1 bis 5,5 % Fettsäureglucamid gemäß e).

**3.** Zusammensetzung nach Anspruch 1 oder 2, wobei das mindestens eine Fettsäureglucamid folgende allgemeine Formel hat:

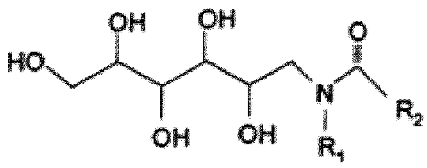

(1)

bei der:

$R_1$ = H oder ein C1- bis C4-Alkyl ist,
$R_2$ = ein C6 bis C20 geradkettiger oder verzweigter, gesättigter oder ungesättigter Kohlenwasserstoffrest ist, wobei vorzugsweise $R_1$ gleich -H oder -$CH_3$, noch bevorzugter =$CH_3$ ist.

**4.** Zusammensetzung nach Anspruch 3, bei der $R_2$ ein C8 bis C18 geradkettiger, verzweigter oder unverzweigter, gesättigter oder ungesättigter Kohlenwasserstoffrest ist.

**5.** Zusammensetzung nach den Ansprüchen 1 bis 4, bei der das Glucamid gemäß e) eine Mischung umfasst, die aus Capryloyl/Caproyl-Methylglucamid, Lauroyl/Myristoyl-Methylglucamid und Cocoyl-Methylglucamid ausgewählt ist, wobei das Glucamid gemäß e) vorzugsweise Cocoyl-Methylglucamid ist.

**6.** Zusammensetzung nach den Ansprüchen 1 bis 5, bei der das Öl gemäß b) ein Isopropyl- oder Ethylhexylfettsäureester oder Alkylbenzoat ist, wobei das Öl gemäß b) vorzugsweise aus Ethylhexylstearat, Isopropylmyristat, Isopropylpalmitat, Isopropylstearat und Isopropylestern von Rapsöl oder Mischungen davon ausgewählt ist.

**7.** Zusammensetzung nach den Ansprüchen 1 bis 6, bei der die Zusammensetzung 1 bis 3 Gew.-% des Öls gemäß b) umfasst.

**8.** Zusammensetzung nach Anspruch 1, wobei der Schaumbildner ausgewählt ist aus Cocamidopropylbetain, Capryl/Capamidopropylbetain, Fettalkoholethercarbonsäuren und -carboxylaten, Alkanolamiden, Aminoxiden und ethoxylierten Alkylsulfosuccinaten.

**9.** Zusammensetzung nach den Ansprüchen 1 bis 8, umfassend 1 bis 3 Gew.-% des Emulgators gemäß a).

**10.** Zusammensetzung nach den Ansprüchen 1 bis 9, umfassend PEG-7-Glycerylcocoat, Polyglyceryl-3-Caprinat, Polyglycerin-4-Caprinat, Glycereth-2-Cocoat oder PEG-6-Capryl-/Caprinsäureglyceride als der Weichmacher gemäß c).

**11.** Zusammensetzung nach den Ansprüchen 1 bis 10, bei der die Zusammensetzung 8 bis 15% anionisches Tensid gemäß d) umfasst.

**12.** Zusammensetzung nach den Ansprüchen 1 bis 11, umfassend 2 bis 5 Gew.-% des Schaumbildners gemäß f).

**13.** Zusammensetzung nach den Ansprüchen 1 bis 12, bei der die waschaktiven Substanzen 7 bis 20 % des Gesamtgewichts der Zusammensetzung ausmachen.

**14.** Verwendung einer Zusammensetzung nach den Ansprüchen 1 bis 13 zur Reinigung von Haut und/oder Händen.

**15.** Überkopf-Schaumspender, umfassend eine Zusammensetzung nach den Ansprüchen 1 bis 14.

**16.** Verfahren zur Herstellung eines Schaums, umfassend das Bereitstellen einer Zusammensetzung nach den Ansprüchen 1 bis 14 in einem Überkopf-Schaumspender und den Betrieb des Spenders zur Erzeugung des Schaums.

## Revendications

**1.** Composition nettoyante moussante pour la peau et les mains comprenant les composants suivants, dans chaque cas basés sur la composition totale de la composition nettoyante :

a) 0,1 à 5 % en poids d'au moins un émulsionnant choisi parmi le dioléate de ricin PEG-18, le 4-caproate de polyglycérol, l'huile de ricin hydrogénée, le dioléate de glycéryle PEG-10, le caprylate/caprate de glycéreth-7, les tensioactifs jumelés et leurs mélanges ;
b) 0,5 à 7 % en poids d'au moins une huile, en particulier d'un ester alkylique d'acide gras ;
c) 0 à 10 % en poids d'au moins un émollient hydrophobe ; choisi parmi les esters de polyol, et typiquement choisis parmi le (coprah-yl)ate de glycéryle PEG7, le 4-caprate de polyglycérol, le 2-(coprah-yl)ate de glycéreth, les glycérides capryliques/capriques de PEG-6, le 3-caprate de polyglycéryle, le 4-caprate de polyglycéryle et leurs mélanges ;
d) 5 à 20 % en poids d'au moins un tensioactif utilisé en tant qu'agent d'enlèvement des salissures ;
e) 0,5 à 12 % en poids d'au moins un glucamide d'acide gras ;
f) 1 à 8 % en poids d'au moins un tensioactif utilisé en tant qu'agent moussant ;
g) 0 à 1 % en poids d'au moins un conservateur ;
h) 0 à 1 % en poids d'un modificateur de pH ;
i) 0 à 10 % en poids d'un ou plusieurs auxiliaires ou additifs ;
j) de l'eau pour atteindre 100 % en poids.

**2.** Composition selon la revendication 1, comprenant 1 à 5,5 % de glucamide d'acide gras (e).

**3.** Composition selon la revendication 1 ou 2, dans lequel l'au moins un glucamide d'acide gras est de formule générale :

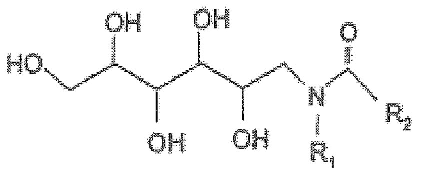

(1)

dans laquelle :

$R^1$ = H ou un alkyle en $C_1$ à $C_4$
$R^2$ = un résidu hydrocarboné en $C_6$ à $C_{20}$ linéaire ou ramifié, saturé ou insaturé,
de préférence dans laquelle $R_1$ est -H ou $-CH_3$, plus préférablement $=CH_3$.

**4.** Composition selon la revendication 3, dans laquelle $R_2$ est un résidu hydrocarboné en $C_8$ à $C_{10}$ linéaire, ramifié ou non ramifié, saturé ou insaturé.

**5.** Composition selon les revendications 1 à 4, dans laquelle le glucamide (e) comprend un mélange choisi parmi le méthyl-glucamide de capryloyle/caproyle, le méthyl-glucamide de lauroyle/myristoyle ; et le méthyl-glucamide de coprah-yle, le plus préférablement dans laquelle le glucamide (e) est le méthyl-glucamide de coprah-yle.

**6.** Composition selon les revendications 1 à 5, dans laquelle l'huile (b) est un alkylbenzoate ou un ester d'acide gras isopropylique ou éthylhexylique, de préférence dans laquelle l'huile (b) est choisie parmi le stéarate d'éthylhexyle, le myristate d'isopropyle, le palmitate d'isopropyle, le stéarate d'isopropyle et les esters isopropyliques d'huile de colza ou leurs mélanges.

**7.** Composition selon les revendications 1 à 6, dans laquelle la composition comprend 1 à 3 % en poids de l'huile (b) .

**8.** Composition selon la revendication 1, dans laquelle l'agent moussant est choisi parmi la (coprahyl)amidopropyl-bétaine, la capryl/capramidopropyl-bétaïne, les acides carboxyliques et carboxylates d'éther et d'alcool gras, les alcanolamides, les oxydes d'amine et les alkylsulfosuccinates éthoxylés.

**9.** Composition selon les revendications 1 à 8, comprenant 1 à 3 % en poids de l'émulsionnant (a).

**10.** Composition selon les revendications 1 à 9, comprenant du (coprah-yl)ate de glycéryle PEG-7, du 3-caprate de polyglycéryle, du 4-caprate de polyglycérol, du (coprah-yl)ate de glycéreth-2, ou des glycérides capryliques/capriques PEG-6 en tant qu'émollient (c).

**11.** Composition selon les revendications 1 à 10, dans laquelle la composition comprend 8 à 15 % de tensioactif anionique

(d).

**12.** Composition selon les revendications 1 à 11, comprenant 2 à 5 % en poids d'agent moussant (f).

**13.** Composition selon les revendications 1 à 12, dans laquelle les substances actives de lavage représentent 7 à 20 % du poids total de la composition.

**14.** Utilisation d'une composition selon les revendications 1 à 13 pour le lavage de la peau et/ou des mains.

**15.** Distributeur de mousse inversé comprenant une composition selon les revendications 1 à 14.

**16.** Procédé pour produire une mousse, comprenant la disposition d'une composition selon les revendications 1 à 14 dans un distributeur de mousse inversé et l'actionnement du distributeur pour qu'il génère la mousse.

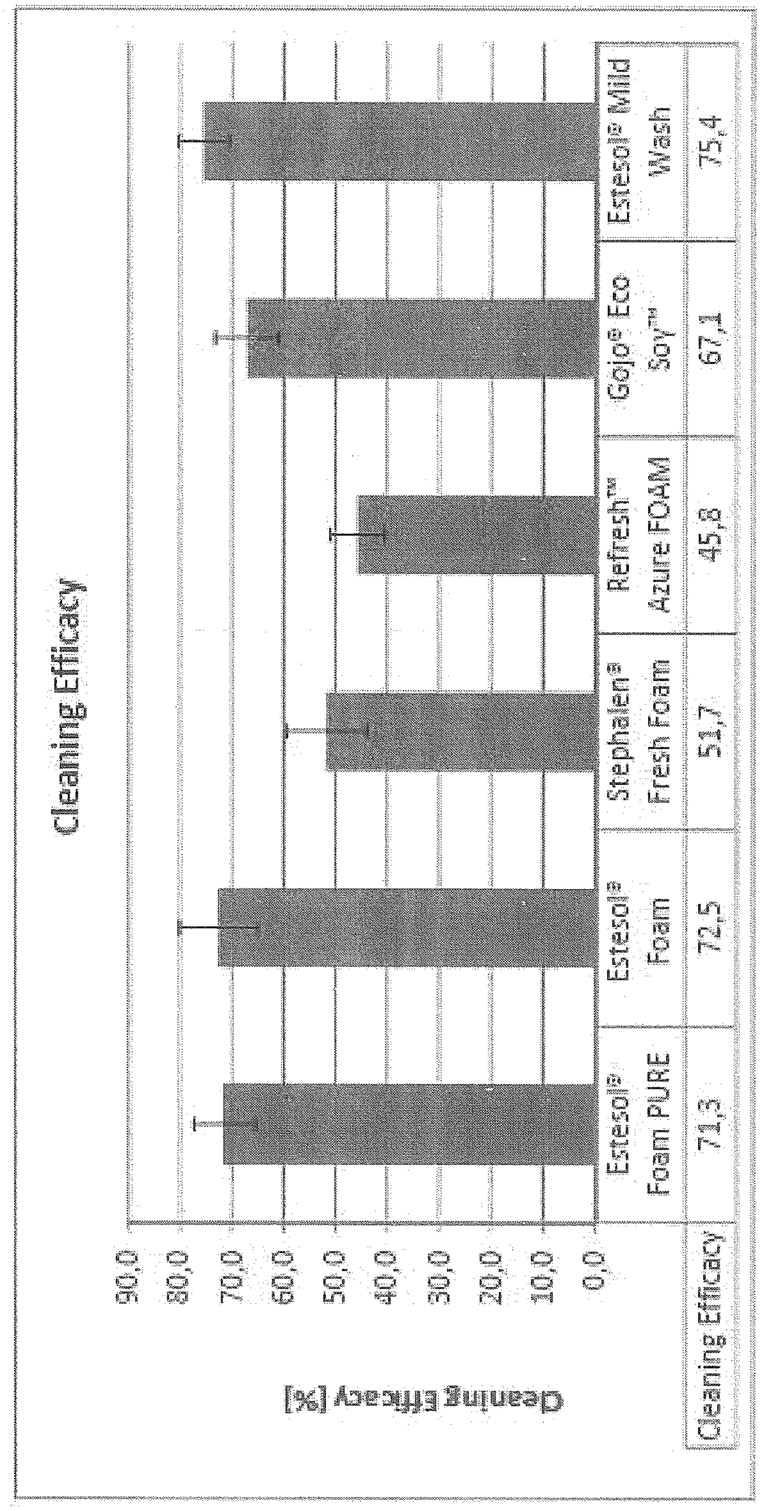
Cleaning Efficacy
Cleaning Efficacy [%]
90,0
80,0
70,0
60,0
50,0
40,0
30,0
20,0
10,0
0,0
Cleaning Efficacy
Estesol® Foam PURE
71,3
Estesol® Foam
72,5
Stephalen® Fresh Foam
51,7
Refresh™ Azure FOAM
45,8
Gojo® Eco Soy™
67,1
Estesol® Mild Wash
75,4

Figure 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9949769 A
- WO 2014138958 A
- WO 2012021130 A
- EP 1072615 A

### Non-patent literature cited in the description


- **S. K. HATE ; S.P. MOULIK.** *Current Science,* 2002, vol. 82 (9), 1101-1111